# EUROPEAN PATENT APPLICATION

(11) **EP 2 829 182 A2**
(43) Date of publication of application: **28.01.2015**
(21) Application number: 14171299.2
(22) Date of filing: 05.06.2014
(51) Int. Cl.: A23L 1/052, A23L 1/0522, A23L 1/053, A23L 1/054, A23L 1/09

(54) **Novel thickening composition comprising pregelatinized waxy potato starch or pregelatinized cassava starch**

(30) Priority: 22.07.2013 US 201313947261
(71) Applicant: Corn Products Development, Inc, 04311-000SP Sao Paulo (BR)
(72) Inventor: Skorge, Robert A., Somerville, NJ08876 (US); Sekula, Bernard C., Glen Gardner, NJ 08826 (US); Staniszewski, Paul, Newark, NJ 19711 (US); Maloney, Sean, Warrington, PA 18976 (US)
(74) Representative: Held, Stephan

(57) **Abstract**

The application relates to a composition comprising from about 10% (w/w) to about 40% (w/w) pregelatinized waxy potato starch, pregelatinized waxy cassava starch, or any mixture thereof; from about 10% (w/w) to about 20% (w/w) gum; and from about 40% (w/w) to about 70% (w/w) dispersant.

## Description

### INTRODUCTION

Dysphagia is a condition in which individuals have difficulty with food mastication and swallowing. While there are a variety of causes of dysphagia and varying levels of affliction, swallowing difficulties can result in aspiration of thin liquids and choking on solid foods. Swallowing difficulties often discourage fluid or food intake leading to concerns about adequate hydration and nutrition in the diets of dysphagic patients. Textural modification, such as food purees and thickened beverages have been employed to facilitate "safe" swallowing.

Commercial thickening agents are typically used to sufficiently thicken beverages so that the fluids can be safely consumed by individuals with dysphagia. Commercially available thickeners used currently for dysphagia all exhibit significant drawbacks that negatively affect acceptability and compliance in patient use. Liquids thickened with starches (*e.g.* modified corn starch) are opaque and have a grainy and/or pulpy texture. They also have a "starchy" flavor. Liquids thickened with gums *(e.g.* xanthan gum) have a gloppy and/or cohesive texture and a slippery and/or slimy mouth feel. Thickened liquid products exhibiting textural and sensorial attributes reminiscent of the non-thickened versions would improve acceptability and patient compliance thereby promoting adequate hydration and nutrition.

### SUMMARY

In one aspect, the application provides a composition comprising from about 10% (w/w) to about 40% (w/w) pregelatinized waxy potato starch, pregelatinized waxy cassava starch, or any mixture thereof; from about 10% (w/w) to about 20% (w/w) gum; and from about 40% (w/w) to about 70% (w/w) dispersant.

### BRIEF DESCRIPTION OF THE DRAWINGS

Fig. 1 depicts the batch agglomeration process of the present application.
Fig. 2 depicts the viscosity as a function of concentration of Example 1 (Xanthan Gum:Waxy Potato:Maltodextrin 17.3:19.3:63.4) and of two commercial thickeners.

### DETAILED DESCRIPTION

A novel thickening composition has been developed that mitigates the negative drawbacks of starch- and gum-only thickening agents. The components of the composition and their blend ratios are selected based on their viscosifying behavior across a range of liquid beverages, and their low turbidity in water. The starch and gum components may be dry blended alone, or dry blended with some of the entire dispersant, or dry blended with the entire dispersant prior to agglomeration. Additionally the dry components may be added separately to the agglomerator using the fluidizing air to help blend the materials during agglomeration. The dispersant may be added as part of the dry blend or may be added to the binding water used in agglomeration. The starch may be native or modified by chemical or physical means. In one embodiment the starch is pregelatinized waxy potato starch, pregelatinized waxy cassava starch, or any mixtures thereof. The novel composition blend is agglomerated to facilitate rapid dispersability and hydration.

In one aspect, the application provides a composition comprising from about 10% to about 40% starch, from about 10% to 20% gum, and from about 40% to about 70% dispersant (w/w).

In one aspect, the application provides a composition comprising from about 10% (w/w) to about 40% (w/w) pregelatinized waxy potato starch, pregelatinized waxy cassava starch, or any mixture thereof; from about 10% (w/w) to about 20% (w/w) gum; and from about 40% (w/w) to about 70% (w/w) dispersant.

In one embodiment, the composition comprises from about 15% (w/w) to about 35% (w/w) pregelatinized waxy potato starch, pregelatinized waxy cassava starch, or any mixture thereof; from about 12% (w/w) to about 18% (w/w) gum; and from about 50% (w/w) to about 60% (w/w) dispersant.

In one embodiment, the composition comprises from about 15% (w/w) to about 35% (w/w) pregelatinized waxy potato starch, pregelatinized waxy cassava starch, or any mixture thereof; from about 12% (w/w) to about 18% (w/w) gum; and from about 40% (w/w) to about 70% (w/w) dispersant.

In one embodiment, the composition comprises from about 15% (w/w) to about 35% (w/w) pregelatinized waxy potato starch, pregelatinized waxy cassava starch, or any mixture thereof; from about 14% (w/w) to about 16% (w/w) gum; and from about 53% (w/w) to about 57% (w/w) dispersant.

In one embodiment, the composition comprises from about 28% (w/w) to about 32% (w/w) pregelatinized waxy potato starch, pregelatinized waxy cassava starch, or any mixture thereof; from about 14% (w/w) to about 16% (w/w) gum; and from about 53% (w/w) to about 57% (w/w) dispersant.

In one embodiment, the gum is clarified xanthan gum.

In one embodiment, the dispersant is maltodextrin.

In one embodiment, the maltodextrin has a DE from about 20 to about 10.

In one embodiment, the maltodextrin has a DE from about 19 to about 14.

In one embodiment, the maltodextrin has a DE of about 18.

In one embodiment, the product is agglomerated.

In one embodiment, the ratio of dispersant to that of the sum of the starch and gum is from about 1:1 (w/w) to about 1:0.42 (w/w).

In one embodiment, the measured turbidity of an aqueous solution with a Brookfield viscosity of about 1000 cP is less than 150 NTU, when the Brookfield viscosity is measured by the Brookfield Viscosity Evaluation Procedure of the Examples.

In one embodiment, the pregelatinized waxy potato starch, pregelatinized waxy cassava starch, or any mixture thereof is native pregelatinized waxy potato starch, native pregelatinized waxy cassava starch, or any mixture thereof.

In one embodiment, the ratio of pregelatinized waxy potato starch, pregelatinized waxy cassava starch, or any mixture thereof to gum is from about 1:2 (w/w) to about 1:0.5 (w/w).

In another aspect, the application provides a food product, said food product comprising from about 10% (w/w) to about 40% (w/w) pregelatinized waxy potato starch, pregelatinized waxy cassava starch, or any mixture thereof; from about 10% (w/w) to about 20% (w/w) gum; and from about 40% (w/w) to about 70% (w/w) dispersant; and any other edible ingredient.

In one embodiment, the food product is a beverage.

In one aspect, the application provides a method of making a composition comprising from about 10% (w/w) to about 40% (w/w) pregelatinized waxy potato starch, pregelatinized waxy cassava starch, or any mixture thereof; from about 10% (w/w) to about 20% (w/w) gum; and from about 40% (w/w) to about 70% (w/w) dispersant comprising blending said starch, said gum, and said dispersant in any order.

In one aspect, the application provides a method of treating dysphagia with a food product, said food product comprising from about 10% (w/w) to about 40% (w/w) pregelatinized waxy potato starch, pregelatinized waxy cassava starch, or any mixture thereof; from about 10% (w/w) to about 20% (w/w) gum; and from about 40% (w/w) to about 70% (w/w) dispersant; and any other edible ingredient.

In one aspect, the application provides a composition comprises from about 10% (w/w) to about 40% (w/w) pregelatinized waxy potato starch, pregelatinized waxy cassava starch, or any mixture thereof; from about 10% (w/w) to about 25% (w/w) gum; and from about 50% (w/w) to about 80% (w/w) dispersant.

In one embodiment, the application provides the composition comprising from about 20% (w/w) to about 25% (w/w) pregelatinized waxy potato starch, pregelatinized waxy cassava starch, or any mixture thereof; from about 15% (w/w) to about 20% (w/w) gum; and from about 55% (w/w) to about 65% (w/w) dispersant.

In one aspect, , the application provides a composition comprising from about 8% (w/w) to about 12% (w/w) pregelatinized waxy potato starch, pregelatinized waxy cassava starch, or any mixture thereof; from about 18% (w/w) to about 22% (w/w) gum; and from about 65% (w/w) to about 75% (w/w) dispersant.

This agglomerated blend allows for rapid dispersability as well as a rapid build and more consistent maintenance of viscosity across a range of liquid beverages. The beverages thickened via the novel composition retained more of their natural, non-thickened attributes. Addition of the novel thickening composition to clear liquids results in thickened products that exhibit low opacity, smooth appearance, little or no cling ('legs') to the sides of a glass, and no perceived aroma or flavor. Unexpectedly, the viscosity of liquids thickened with this novel composition was perceived to be lower than liquids thickened with commercially available thickening agents despite the fact that the actual measured viscosities were equivalent.

### DEFINITIONS

The following definitions are used in connection with the compounds of the present application unless the context indicates otherwise. The abbreviation "CMC" means carboxymethylcellulose. The abbreviation "DE" means Dextrose Equivalent. "DE" is defined as the reducing power of starch substance. Each starch molecule has one reducing end; therefore DE is inversely related to molecular weight. The DE of anhydrous D-glucose is defined as 100 and the DE of unhydrolyzed starch is virtually zero. The abbreviation "NTU" means nephlelometric turbidity unit. The abbreviation "w/w" means weight per weight. As used herein, the term "agglomerate" refers to several particles adhering together.

As used herein, the term "dispersant" refers to a substance, usually a colloid, that when added to a liquid suspension improves the separation of particles in the suspension and prevents self-associated clumping or settling. "Dispersants" include, but are not limited to, sugar derivates like xylitol, erythritol, sorbitol, or mannitol; monosaccharides like glucose, mannose, fructose, or galactose; disaccharides like sucrose, maltose, or lactose; oligosaccharides like maltodextrin; polyethyleneglycol; glycerol; triacetin; glycofurol; effervescents; or any mixtures thereof.

As used herein, the term "gelatinized" starch refers to swollen starch granules which have lost their polarization crosses and which may or may not have lost their granular structure. When starches are heated in the presence of water above their gelatinization temperature the birefringence of the granules is irreversibly destroyed during the hydration and the starch molecules will become highly soluble. The thermal processes used to gelatinize starches include batch cooking, autoclaving, and continuous cooking processes, in equipment which includes, without limitation, in a heat exchanger, jet-cooker, spray drier, and drum drier.

As used herein, the term "granular starch" refers to starch which has substantially retained its native granules and are not (pre) gelatinized (ungelatinized).

As used herein, the term "gum" refers to naturally occurring resinous materials from vegetative species and modifications of those naturally occurring materials. Gums that may be used are well known in the art and include xanthan, carrageenan, gellan, locust bean, alginate, pectin, agar, gum Arabic, and guar gum. Materials derived from gums include those listed which have been further modified using methods known in the art such as hydrolysis and chemical modification.

As used herein, the phrase "native starch" means a starch as derived from a plant as it is found in nature. Typical sources for the starches are cereals, tubers and roots, legumes and fruits. The native source can be any variety, including without limitation, corn, potato, sweet potato, barley, wheat, rice, sago, amaranth, cassava (tapioca), arrowroot, canna, pea, banana, oat, rye, triticale, and sorghum, as well as low amylose (waxy) and high amylose varieties thereof. As used herein, the term "pregelatinized" refers to a dry starch, which has been gelatinized and dried prior to being used for its intended purpose. "Pregelatinized" starch, also known as cold water soluble (CWS) or dispersed starch has swollen starch particles and has lost its birefringence and/or Maltese crosses in polarized light. Such a "pregelatinized" starch is substantially soluble in cold water without cooking. In this context "soluble" does not necessarily mean the formation of a true molecular solution, but may also mean a colloidal dispersion. In one embodiment, the starch is completely "pregelatinized".

The "pregelatinized" starches" are well known in the art, for example in U.S. Pat. Nos. 4,465,702, 5,037,929, 5,131,953, and 5,149,799, the disclosures of which are incorporated by reference in their entirety. Methods of preparing "pregelatinized" starches by thermal, chemical or mechanical gelatinization and then drying are well known in the art. The thermal processes used to prepare pregelatinized starches include batch cooking, autoclaving, and continuous cooking processes in a heat exchanger or jet-cooker. Methods of preparing "pregelatinized" starches are also described in such articles as Chapter XXII-- "Production and Use of Pregelatinized Starch" Starch: Chemistry and Technology, Vol. III--Industrial Aspects, R. L. Whistler and E. F. Paschall, Editors, Academic Press, New York 1967, incorporated by reference in its entirety. Another method of pregelatinizing starch while maintaining the granular structure is by steam injected dual atomization process. This process is known in the art and is exemplified in U.S. Patent Nos. 4,280,851 and 4,600,472, the disclosures of which are incorporated by reference in their entirety.

As used herein, the term "waxy" is intended to mean a starch containing less than 10% amylose by weight, in one embodiment less than 5% amylose by weight, in another embodiment less than 2% amylose by weight, and in yet another embodiment less than 1% amylose by weight of the starch. Typical sources for the waxy starches are cereals, tubers, roots, legumes and fruits. The source can be corn, pea, potato, sweet potato, banana, barley, wheat, rice, sago, amaranth, cassava, arrowroot, canna, and sorghum. Particularly useful waxy starches are waxy starches, including waxy maize, waxy cassava, waxy pea, waxy wheat, waxy rice, waxy potato, waxy sorghum, and waxy barley.

A "waxy cassava starch" has an amylose content of less than 4% or only trace amounts (less than 3%), depending whether it is measured by colorimetric or DSC methods respectively. Normal cassava starch has an amylose content of about 21% or about 19%, depending whether it is measured by colorimetric or DSC methods respectively. Waxy cassava starch was recently described by Sánchez T., Dufour D., Moreno I.X., Ceballos H., 2010, "Comparison of pasting and gel stabilities of waxy and normal starches from potato, maize, and rice with those of a novel waxy cassava starch under thermal, chemical, and mechanical stress", Journal of Agricultural and Food Chemistry, 58: 5093-5099, which is incorporated by reference in its entirety.

A "waxy potato starch" has an amylose content of less than 4% measured by colorimetric methods. Normal potato starch has an amylose content of about 29% measured by colorimetric methods.

Certain specific aspects and embodiments of the present application will be explained in greater detail with reference to the following Embodiments and Examples, which are provided only for purposes of illustration and should not be construed as limiting the scope of the application in any manner. Reasonable variations of the described procedures are intended to be within the scope of the present invention. While particular aspects of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

### EMBODIMENTS

1. A composition comprising from about 10% (w/w) to about 40% (w/w) pregelatinized waxy potato starch, pregelatinized waxy cassava starch, or any mixture thereof; from about 10% (w/w) to about 20% (w/w) gum; and from about 40% (w/w) to about 70% (w/w) dispersant.
2. The composition of embodiment 1 comprising from about 15% (w/w) to about 35% (w/w) pregelatinized waxy potato starch, pregelatinized waxy cassava starch, or any mixture thereof; from about 12% (w/w) to about 18% (w/w) gum; and from about 50% (w/w) to about 60% (w/w) dispersant.
3. The composition of embodiment 1 comprising from about 28% (w/w) to about 32% (w/w) pregelatinized waxy potato starch, pregelatinized waxy cassava starch, or any mixture thereof; from about 14% (w/w) to about 16% (w/w) gum; and from about 53% (w/w) to about 57% (w/w) dispersant.
4. The composition of embodiment 1 comprising from about 20% (w/w) to about 25% (w/w) pregelatinized waxy potato starch, pregelatinized waxy cassava starch, or any mixture thereof; from about 15% (w/w) to about 20% (w/w) gum; and from about 55% (w/w) to about 65% (w/w) dispersant.
5. The composition of embodiment 1, wherein the gum is clarified xanthan gum.
6. The composition of embodiment 1, wherein the dispersant is maltodextrin.
7. The composition of embodiment 6, wherein the maltodextrin has a DE from about 20 to about 10.
8. The composition of embodiment 7, wherein the maltodextrin has a DE of about 18.
9. The composition of embodiment 1, wherein the product is agglomerated.
10. The composition of embodiment 1, wherein the ratio of dispersant to that of the sum of the starch and gum is from about 1:1 (w/w) to about 1:0.42 (w/w).
12. The composition of embodiment 1, wherein the measured turbidity of an aqueous solution with a Brookfield viscosity of about 1000 cP is less than 150 NTU, when the Brookfield viscosity is measured by the Brookfield Viscosity Evaluation Procedure.
13. The composition of embodiment 1, wherein the pregelatinized waxy potato starch, pregelatinized waxy cassava starch, or any mixture thereof is native pregelatinized waxy potato starch, native pregelatinized waxy cassava starch, or any mixture thereof.
14. The composition of embodiment 1, wherein the ratio of pregelatinized waxy potato starch, pregelatinized waxy cassava starch, or any mixture thereof to gum is from about 1:2 (w/w) to about 1:0.5 (w/w).
15. A food product, said food product comprising the composition of embodiment 1 and any other edible ingredient.
16. The food product of embodiment 15, wherein the food product is a beverage.
17. A method of making the composition of embodiment 1 comprising blending the starch, the gum, and the dispersant in any order.
18. A method of treating dysphagia with the food product of embodiment 15.

### EXAMPLES

All parts and percentages are given by weight and all temperatures in degrees Celsius (°C) unless otherwise noted. The following analytical procedures were used throughout the examples.

Transmittance Evaluation Procedure. The light transmittances of samples were assessed at 600 nm using a spectrophotometer. Transmittance of the solution was determined by the ratio of light transmitted by the composition solution at the indicated composition to that of the pure water. A 2% difference in transmittance is noticeable to the human eye.

Turbidity Evaluation Procedure. A Hach 2100Q Portable Turbidimeter was used to measure the turbidity of the samples. The instrument was calibrated using 20, 100 and 800 NTU standards prior to making any sample measurements.

**Particle Size Measurement Procedure.** The particle size and distribution of the agglomerated samples were determined using a RO-TAP^{®} sieve shaker. Testing involved stacking in decreasing order a set of sieves of various sizes on top of each other with the larger-hole sieves are on top and smaller-hole sieves on the bottom; a solid pan was at the bottom to catch sample particles passing through the smallest sieve opening. The set of sieves was placed on a RO-TAP^{®} sieve shaker which mechanically shakes in a circular motion while tapping the top of the stack and causes various sized particles to move through the stack of sieves until they rest on a sieve whose openings are too small for them to pass through. After a designated period of time the sieves were removed from the RO-TAP^{®} sieve shaker and each sieve's contents were weighed and recorded. The percentage retained on each sieve was used to classify the size fractions present in the sample.

**Brookfield Viscosity Evaluation Procedure.** The viscosity and appearance of samples were assessed using a Brookfield Model LV2 Model LVTDV apparatus at ambient temperature (25 °C.) with a spindle #3 at 60 rpm for 10 seconds or at a constant shear rate of 50 s-1 with a SSA #21 spindle as recommended by the National Dysphagia Diet Task Force. A viscosity reading of >2000 indicate that the viscosity is beyond the capability of the instrument to accurately measure. **Bostwick Consistency Evaluation Procedure.** The viscosity and consistency of each sample was measured using a Bostwick consistometer and the standard methodology described in 21 CFR §155.194(b). The temperature of the sample was adjusted to 20 ± 1 °C. as was the Bostwick trough. The end-to-end level of the Bostwick consistometer was adjusted by means of the spirit level placed in the trough of the instrument. The side-to-side level was adjusted by means of the built-in spirit level. The sample was then transferred to the dry sample chamber of the Bostwick consistometer, filling the chamber slightly more than level full and avoiding air bubbles. A straight edge was passed across the top of the chamber starting from the gate end to remove excess sample. The gate was released by gradual pressure on the lever, holding the instrument down at the same time to prevent movement as the gate was released. After 30 seconds, the maximum distance of flow was read.

**Component Evaluation and Selection.** Various pregelatinized starches and gums were combined and evaluated at a gum:starch:maltodextrin ratio of 5:50:45 (w/w). Six and one-half percent solutions were prepared with bottled water. The Brookfield viscosity and turbidity of the fully dispersed and hydrated samples were measured. The results are shown in Table 1. Waxy potato starch provided good viscosity with lower turbidity than waxy corn starch, modified potato starch, or previously agglomerated waxy potato starch. Similarly clarified xanthan gum provided lower turbidity than standard xanthan or guar gums. Although CMC provided low turbidity when combined with waxy potato starch, its performance was not as good as clarified xanthan gum across a number of beverage applications.

**Table 1**

| Starch | | | Gum | | Viscosity (cP) | Turbidity (NTU) |
|---|---|---|---|---|---|---|
| Source | Modified | Agglomerated | Source | Comment | | |
| Waxy Corn | No | No | xanthan | standard | 802 | > 1000 |
| Waxy Corn | No | No | xanthan | clarified | 808 | < 1000 |
| Waxy Potato | No | No | xanthan | standard | 946 | 130 |
| Waxy Potato | No | No | xanthan | clarified | 1073 | 73 |
| Waxy Potato | No | No | CMC | standard | 805 | 76 |
| Waxy Potato | No | No | Guar | standard | 860 | 359 |
| Waxy Potato | No | Yes | xanthan | clarified | 1723 | 293 |
| Potato | Yes | No | xanthan | clarified | 2523 | > 1000 |

### Batch Agglomeration Process Example 1 (Xanthan Gum:Waxy

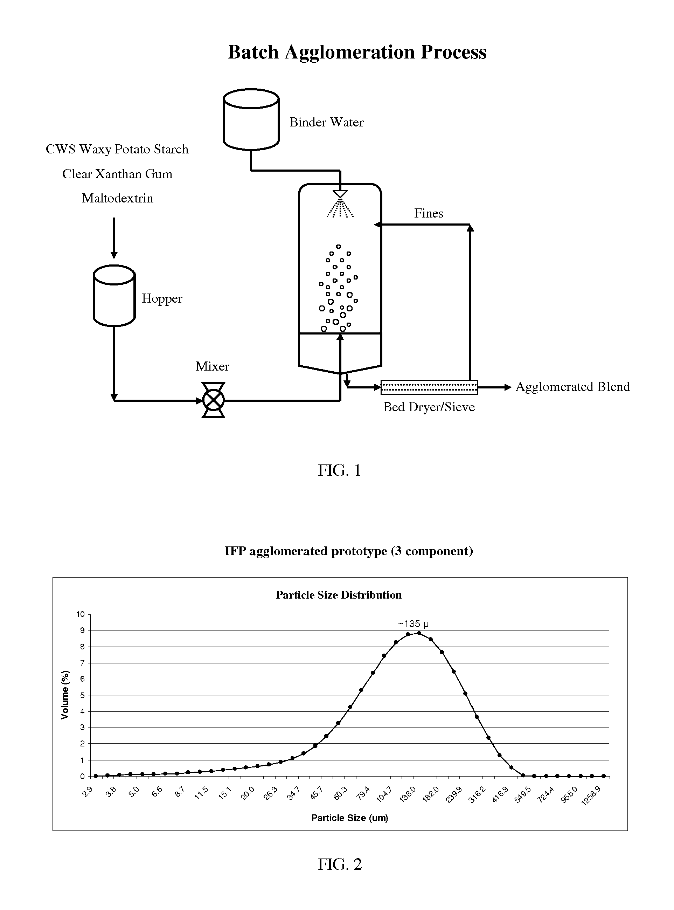

**Potato:Maltodextrin 17.3:19.3:63.4).** The novel composition blend with waxy potato starch was agglomerated to facilitate rapid dispersability and hydration. The batch agglomeration process is shown Fig. 1. A mixture of cold water soluble waxy potato starch (381 g), clear xanthan gum (341 g), and maltodextrin (837 g of 18DE) was added to the bowl of a fluid bed granulation system. The bowl was sealed within the fluid bed system and airflow through the system was initiated at 24 L/sec. As the air mixed the dry powders introduced into the bowl, the air was heated with a closed-loop electric air heater to a desired set point of 70°C. The components in the bowl were allowed to mix and reach an internal temperature of 45°C (after approximately 6 minutes) at which point the addition of a binder solution began. The binder solution was deionized water (837 g) which had been supplemented with maltodextrin (412 g of 18DE) and potassium chloride (4.3 g). This mixture, the binder solution, was then sprayed onto the fluidized bed of powders at a rate of 48 g/min. While the spray was initiated, the flow rate of air through the bed was increased to 38 L/sec and the inlet air temperature was increased to 80 °C. This was done in order to keep the product temperature during the spraying of the binder solution between 41 and 44°C. The spray continued for 26 minutes, after which time the spray source was switched to pure deionized water in order to flush the remaining binder solution in the system tubing into the product. After three minutes of water spray (also at a rate of 48 g/min), the spraying was stopped. At this point, the product continued to be fluidized by air (38 L/sec, 80°C) to remove some of the excess moisture from the product. After two minutes, when the product temperature reached 45°C, the electric air heating was turned off, after which the air flow to the fluidized bed was turn off. At this point, the bowl was removed from the granulator and the product was recovered after removing oversized pieces by passing it through a 1.3 mm wire opening sieve. The characteristic moisture of the product after this process was five to seven percent by weight (measured by loss on drying).

Viscosity curves were developed for the agglomerated prototype as well as for commercial starch-based and xanthan gum-based thickeners. As seen in Figure 2 the agglomerated prototype exhibited smaller changes in viscosity with increasing concentration suggesting it has greater dosing flexibility under conditions of misuse, *i.e.* less likelihood of missing the desired viscosity when over- or under-dosing occurs. The distribution of particle sizes of the agglomerated composition is shown in Table 2. The bulk density of the agglomerated composition was between 0.25 and 0.30 g/cm³.

The prototype (Example 1, Xanthan Gum:Waxy Potato:Maltodextrin 17.3:19.3:63.4) and several commercial products were also evaluated as blind coded samples in a clinical setting by a dysphagia expert. Products were added at the manufacturer's prescribed usage levels to thicken water to a nectar consistency (350 to 500 cPs). Their consistency (resistance to flow) was measured using a Bostwick consistometer. The thickened liquids were also evaluated via a 15 point scale during preparation and after thickening. The attributes, attribute ranges and ratings for the various samples are provided in Tables 3 and 4. Despite being close in measured viscosity, water thickened using the agglomerated composition exhibited the highest flow (*i.e.* highest Bostwick value) and the lowest perceived viscosity. The overall (sensorial) preference of the prototype was higher than the commercial products suggesting that the prototype's unique composition and/or blend ratio minimized the drawbacks of starch-only or gum-only products.

**Table 2**

| | sieve analysis | | | | | | |
|---|---|---|---|---|---|---|---|
| US Mesh | 16 | 20 | 40 | 60 | 80 | 100 | pan |
| microns | 1190 | 841 | 400 | 250 | 177 | 149 | |

| | % retained | | | | | | |
|---|---|---|---|---|---|---|---|
| Batch 1 | 0.4 | 8.4 | 63.6 | 90.3 | 95.6 | 97.1 | 2.9 |
| Batch 2 | 0.9 | 9.3 | 68.9 | 96.7 | 98.1 | 98.6 | 1.4 |

**Table 3**

| In Preparation | | | |
|---|---|---|---|
| Thickener | Dispersibility | Consistency Over Time | Overall Liking |
| Attribute Range the scale is 1 to 15 except as noted | Easy to Difficult | Thins to No Change to Thickens | Hate to Love |
| Modified Starch | 9.6 | 2.0 | 9.0 |
| Example 1 (Xanthan Gum:Waxy Potato: Maltodextrin 17.3:19.3:63.4) | 1.4 | 9.0 | 13.5 |
| Xanthan Gum | 1.0 | 3.0 | 14.0 |
| Carrageenan & Xanthan Gum | 6.5 | 8.0 | 7.5 |

**Table 4**

| Of Finished Product (Thickened Water) | | | | | | | | |
|---|---|---|---|---|---|---|---|---|
| Thickener | Bostwick Consistency Range (cm) | Perceived Thickness | Lumpiness | Clarity | Surface Appear ance | Slipperiness/Sliminess | Perceived Flavor | Overall Preference |
| Attribute Range the scale is 1 to 15 except as noted | 0 to 24 | Thin to Thick | Uniform to Lumpy | Clear to Opaque | Smooth to Pulpy | Hate to Love | None to Oily | Hate to Love |
| Modified Starch | 19.1 | 9.0 | 6.2 | 8.0 | 9.2 | 5.4 | 6.4 | 10.3 |
| Example 1 (Xanthan Gum:Waxy Potato: Maltodextrin 17.3:19.3:63. 4) | 23.5 | 2.2 | 1.6 | 3.8 | 1.4 | 4.0 | 1.0 | 13.5 |
| Xanthan Gum | 16.3 | 8.0 | 1.0 | 1.5 | 1.0 | 10.0 | 1.0 | 10.5 |
| Carrageenan & Xanthan Gum | 20.2 | 4.5 | 3.0 | 1.0 | 1.0 | 3.0 | 1.0 | 7.5 |

Following manufacturer's directions for nectar consistency, the prototype of Example 1 (Xanthan Gum:Waxy Potato:Maltodextrin 17.3:19.3:63.4) and various commercially available products were also evaluated for their ability to thicken and maintain viscosity over 5 hours in various beverage applications as shown in Table 5. On the whole, the prototype outperformed commercially available products.

**Table 5**

| Viscosifying Performance in Applications | | | | | | | | | | | | |
|---|---|---|---|---|---|---|---|---|---|---|---|---|
| Thickener | Cold Water | | Ambient Water | | Cold Orange Juice | | Cold Milk | | Ambient ENSURE^{®} | | Hot or Ambient Coffee | |
| | 30 min | 5 hr. | 30 min | 5 hr. | 30 min | 5 hr. | 30 min | 5 hr. | 30 min | 5 hr. | 30 min | 5 hr. |
| Unthickened | 8 | | | | 26 | | 8 | | 38 | | 2 | |
| Modified Starch | 146 | 193 | 129 | 169 | 925 | 1156 | 301 | 405 | 1253 | ≥ 2000 | 160 | 262 |
| Carrageenan & Xanthan Gum | 395 | 352 | 325 | 321 | 474 | 725 | 144 | 574 | 531 | 1190 | 359 | 461 |
| Xanthan Gum | 91 | 337 | 81 | 339 | 72 | 164 | 33 | 131 | 129 | 339 | 20 | 135 |
| Modified Starch, Xanthan, Tara & Guar Gum | 917 | 1780 | 1245 | 1741 | 685 | 1687 | 66 | 797 | 271 | 1807 | 1150 | 1765 |
| Xanthan & Guar Gum | 789 | 805 | 752 | 677 | 1447 | 1740 | 275 | 988 | 1920 | ≥ 2000 | 1046 | 1114 |
| Xanthan Gum | 638 | 543 | 496 | 425 | 296 | 571 | 43 | 297 | 475 | 1393 | 447 | 543 |
| Example 1 (Xanthan Gum:Waxy Potato: Maltodextrin 17.3:19.3:63.4) | 335 | 296 | 279 | 255 | 362 | 479 | 189 | 266 | 632 | 887 | 252 | 337 |

### Viscosity in cps

**Example 2 with varying starch, gum, and dispersant ratios.** Several blend compositions of clarified xanthan gum, pregelatinized waxy potato starch, and maltodextrin (18DE) were agglomerated using the aforementioned process. Brookfield viscosity, turbidity and transmittance (% trans.) were measured for the agglomerated samples. When dispersed in aqueous solutions all of the agglomerated blends yielded similar viscosities across a range of concentrations as well as relatively low turbidity values as shown in Table 6.

**Table 6**

| Solids Content (%) | Xanthan Gum:Waxy Potato:Maltodextrin Ratio (w/w) | | | | | | | | |
|---|---|---|---|---|---|---|---|---|---|
| | 20:10:70 | | | 17:23:60 | | | 15:30:55 | | |
| | Viscosity (cP) | Turbidity (NTU) | % Trans. | Viscosity (cP) | Turbidity (NTU) | % Trans. | Viscosity (cP) | Turbidity (NTU) | % Trans. |
| 2 | 312 | 17 | 76 | 306 | 23 | 57 | 315 | 39 | 45 |
| 4 | 609 | 26 | 57 | 602 | 30 | 30 | 695 | 55 | 21 |
| 6 | 1100 | 70 | 43 | 1076 | 144 | 24 | 1250 | 103 | 13 |
| 8 | 1752 | 245 | 25 | 1744 | 260 | 13 | >2000 | 237 | 9 |

**Example 3 with waxy potato starch and waxy cassava starch.** The novel composition blends with waxy potato starch or waxy cassava starch were agglomerated to facilitate rapid dispersability and hydration in a similar fashion as in Example 1 (Xanthan Gum:Waxy Potato:Maltodextrin 17.3:19.3:63.4). The Brookfield viscosity and the transmittance of the waxy potato starch containing composition blends and the waxy cassava starch containing composition blends were evaluated at different concentrations using the standard procedures above. The results are shown in Table 7.

**Table 7**

| Description | 2% Conc. | | 4% Conc. | | 6% Conc. | |
|---|---|---|---|---|---|---|
| | visc. (cps) | % trans | visc. (cps) | % trans | visc. (cps) | % trans |
| 15-30-55 WP | 375 | 45 | 830 | 23 | 1510 | 13 |
| 15-30-55 WP | 375 | 43 | 820 | 25 | 1540 | 13 |
| 15-30-55 WC | 390 | 40 | 795 | 19 | 1430 | 11 |
| 15-30-55 WC | 430 | 37 | 835 | 18 | 1685 | 10 |

| | | | | | | |
|---|---|---|---|---|---|---|
| WP = waxy potato starch WC = waxy cassava starch 3 component blend ratio = 15% xanthan gum-30% starch-55% maltodextrin | | | | | | |

**Example 4 with different dispersants.** Maltodextrins with dextrose equivalents of 10, 15, or 18; sucrose; oligofructose; or erythritol were added as dispersants and blended at a gum:starch:dispersant ratio of 15:30:55 (w/w) prior to agglomeration. The agglomerated products exhibited good dispersability and low turbidity (good clarity).

Throughout this application, various publications are referenced. The disclosures of these publications in their entireties are hereby incorporated by reference into this application in order to more fully describe the state of the art as known to those skilled therein as of the date of the application described and claimed herein.

While particular embodiments of the present application have been illustrated and described, it would be obvious to those skilled in the art that various other changes and modifications can be made without departing from the spirit and scope of the application. It is therefore intended to cover in the appended claims all such changes and modifications that are within the scope of this application.

## Claims

1. A composition comprising from about 10% (w/w) to about 40% (w/w) pregelatinized waxy potato starch, pregelatinized waxy cassava starch, or any mixture thereof; from about 10% (w/w) to about 20% (w/w) gum; and from about 40% (w/w) to about 70% (w/w) dispersant.

2. The composition of claim 1 comprising from about 20% (w/w) to about 25% (w/w) pregelatinized waxy potato starch, pregelatinized waxy cassava starch, or any mixture thereof; from about 15% (w/w) to about 20% (w/w) gum; and from about 55% (w/w) to about 65% (w/w) dispersant.

3. The composition of claim 1 or 2, wherein the gum is clarified xanthan gum.

4. The composition of any one of claims 1-3, wherein the dispersant is maltodextrin with a DE from about 20 to about 10.

5. The composition of any one of claims 1-4, wherein the ratio of dispersant to that of the sum of the starch and gum is from about 1:1 (w/w) to about 1:0.42 (w/w).

6. The composition of any one of claims 1-5, wherein the measured turbidity of an aqueous solution with a Brookfield viscosity of about 1000 cP is less than 150 NTU, when the Brookfield viscosity is measured by the Brookfield Viscosity Evaluation Procedure.

7. The composition of any one of claims 1-6, wherein the pregelatinized waxy potato starch, pregelatinized waxy cassava starch, or any mixture thereof is native pregelatinized waxy potato starch, native pregelatinized waxy cassava starch, or any mixture thereof.

8. The composition of any one of claims 1-7, wherein the ratio of pregelatinized waxy potato starch, pregelatinized waxy cassava starch, or any mixture thereof to gum is from about 1:2 (w/w) to about 1:0.5 (w/w).

9. A food product, said food product comprising the composition of any one of claims 1-8 and any other edible ingredient.

10. A method of treating dysphagia with the food product of claim 9.
